# EUROPEAN PATENT APPLICATION

(11) **EP 2 311 788 A1**
(43) Date of publication of application: **20.04.2011**
(21) Application number: 09382207.0
(22) Date of filing: 14.10.2009
(51) Int. Cl.: C07C 51/15, C07C 63/04, C07C 65/21, C07C 65/24, C07C 323/37

(54) **Process for the carboxylation of aryl halides with palladium catalysts**

(71) Applicant: Intitut Català d'Investigació Química (ICIQ), 43007 Tarragona (ES)
(72) Inventor: Martín Romo, Rubén, Francisco, 43007 Tarragona (ES); Correa Navarro, Arkaitz, 43007 Tarragona (ES)
(74) Representative: ZBM Patents

(57) **Abstract**

A process for the carboxylation of an aryl halide to yield an aryl carboxylic acid, in which the aryl halide and CO₂ are contacted in an organic solvent under inert atmosphere and in the presence of a reducing agent and an adequate catalyst system.

## Description

The present invention relates to a process for the carboxylation of aromatic ring systems, namely aryl halides, in order to obtain the corresponding carboxylic acids. Said carboxylic acids are broadly used in the field of pharmaceutical synthesis to get medicinally-important compounds.

### BACKGROUND ART

Because of its abundance, low cost, non-toxicity and high potential as a renewable source, carbon dioxide (CO₂) has recently gained considerable momentum as a source of carbon. Most of the different existing reactions using CO₂ as carbon source are reported by Sakakura et al., in the review "Transformation of Carbon Dioxide", Chemical Reviews - 2007, Vol. 107, No. 6, pp. 2365-2387.

On the other hand, benzoic acid derivatives are motifs found in many natural and medicinally important compounds. Relevant compounds applicable as agricultural chemicals are included in this category too. The obtention of benzoic acid derivatives can be achieved by two powerful alternatives.

One of these alternatives is called metal-catalyzed carbonylation of aryl halides, in which carbon monoxide (CO) is incorporated into a substrate by the addition of CO to an aryl-, benzyl, or vinylpalladium complex in the presence of various nucleophiles. In general, aromatic halides are treated with an appropriate nucleophile in a carbon monoxide atmosphere in the presence of a catalytic amount of a palladium complex, whereby, the leaving group (halogen) is formally replaced by the nucleophiles with incorporation of one or two molecules of carbon monoxide. Said carbonyl reactions are broadly disclosed by Brennführer et al. in "Palladium-catalyzed carbonylation Reactions of Aryl halides and Related Compounds", Angewandte Chemie International Edition - 2009, Vol. 48, pp. 4114-4133. Despite the high yields of the reactions disclosed by Brennführer, the high toxicity associated with CO represents an important issue to be overcome.

Another way to obtain benzoic acid derivatives consists of the carboxylation of carbon nucleophiles with CO₂. This kind of reaction prosecutes through a multi-step process in which an organometallic intermediated species is formed (i.e. Grignard compound). Further, the metallic atom is removed by CO₂ in the presence of a catalyst. Examples of said sort of reactions appear disclosed, for the purpose of example, in the patent document EP 941982 B1. The synthesis of the employed organometallic reagents also represents a drawback for the application of the reactions into industrial processes.

Other multi-step processes for the carboxylation of aromatic compounds are also disclosed in the review of Sakakura et al. (supra), mentioned before, such as the so-called "Kolbe - Schmidt" reaction or the metal-catalyzed reaction of organic halides, CO₂ and hydrogen.

As can be seen, there is a need of further alternative processes, which allow yielding benzoic acid derivatives at the same time the deficiencies (multi-step processes and/or toxicological compounds) of the actually employed industrial processes are overcome.

### SUMMARY OF THE INVENTION

With the aim of improving the pool of reactions to obtain benzoic acid derivatives or, which is the same, carboxylic acids of aryl compounds; and at the meantime retaining in mind the need to preserve the environment by using CO₂ as carbon source, a new process is proposed in which, moreover, the carboxylation reaction is achieved without any toxic reagents and under mild conditions.

Therefore, in a first aspect the invention relates to a process for the carboxylation of an aryl halide comprising contacting the aryl halide with CO₂ under inert atmosphere in an organic solvent and in the presence of a reducing agent and a catalyst system, where the catalyst system comprises a palladium compound and a compound of formula (I) 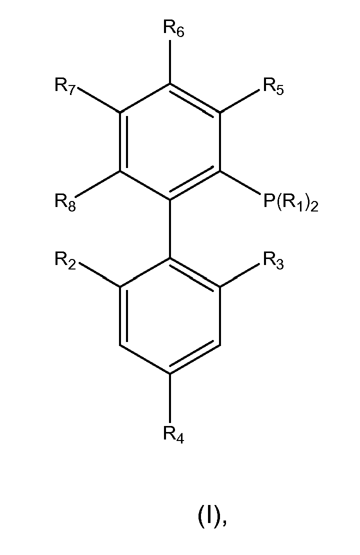 wherein R₁ is a radical independently selected from the group consisting of lineal or branched (C₁-C₆)-alkyl and (C₁-C₁₂)-cycloalkyl;
R₂ and R₃ are radicals independently selected from the group consisting of lineal or branched (C₁-C₆)-alkyl and lineal or branched (C₁-C₆)-alkoxyl;
R₄ is a lineal or branched (C₁-C₆)-alkyl radical;
R₅ is a radical independently selected from the group consisting of H, lineal or branched (C₁-C₆)-alkyl, and lineal or branched (C₁-C₆)-alkoxyl;
R₆, R₇ and R₈ are radicals independently selected from the group consisting of H and lineal or branched (C₁-C₆)-alkyl; and
"aryl" is a C-radical derived from one of the known 1-4 aromatic ring systems, the rings being isolated or partially/totally fused and having 5-6 members; being each member independently selected from C, CH, N, NH, O, and S, being one or more of the hydrogen atoms of said members optionally substituted by radicals selected from the group consisting of: lineal or branched (C₁-C₆)-alkyl optionally substituted by halogen atoms; lineal or branched (C₁-C₆)-alkenyl optionally substituted by an halogen atom; lineal or branched (C₁-C₆)-alkoxyl optionally substituted by an halogen atom; halogen, aldehydes (CHO); ketones of formula CORₐ wherein Rₐ is radical selected from lineal or branched (C₁-C₆)-alkyl and phenyl; amines of formula NRₐR_{b}, wherein Rₐ and R_{b} are lineal or branched (C₁-C₆)-alkyl; ethers of formula ORₑ, wherein Rₑ is a lineal or branched (C₁-C₆)-alkyl or a tetrahydropyranyl group;
thioethers of formula SR_{c} wherein R_{c} is a lineal or branched (C₁-C₆)-alkyl;
esters of formula COOR_{d}, wherein R_{d} is a lineal or branched (C₁-C₆)-alkyl; and epoxides of formula CH₂CHOCHRₐwherein Rₐ is a lineal or branched (C₁-C₆)-al kyl .

The process of the invention can be considered the first disclosed process of direct palladium-catalyzed carboxylation of aryl halides with CO₂. The use of the proposed catalyst system allows the reaction being conducted in such a direct way, being a simple way in comparison with the prior art reactions conducted in multi-step processes. This catalyst system, as disclosed in the PCT patent application WO 2004/052939, has been used in amination reactions and in reaction where an aromatic ring is added to a compound by means of a C-C linkage. The reactions depicted in WO 2004/052939 are of a different nature in respect of the carboxylation process of the present invention. One specific example of amination of aryl chlorides with palladium precatalysts is also disclosed by Christmann et al. in "Experimental and Theoretical Investigations of New Dinuclear Palladium Complexes as Precatalysts for the Amination of Aryl Chlorides", Journal of American Chemical Society - 2006, Vol 128, pp. 6376-6390.

Moreover, the use of the catalyst system comprising a palladium compound and a compound of formula (I), allows the obtention of benzoic acid derivatives using the carbon dioxide as a sole carbon source. As above exposed, this fact is a great goal when thinking in environmental terms.

This and other aspects of the present invention will be further described in the detailed description section that follows, and they are not intended to be limiting of the present invention. Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention.

Furthermore, the present invention covers all possible combinations of particular and preferred groups described hereinabove.

### DETAILED DESCRIPTION OF THE INVENTION

For the purpose of understanding, the following definitions are provided:

As used herein, the term "alkyl" refers to lineal and branched aliphatic hydrocarbon chains of 1 to 6 carbon atoms, as for example, methyl, ethyl, n-propyl, isopropyl, n-butyl, sec-butyl, tert-butyl, isoamyl and n-hexyl.

As used herein, the term "cycloalkyl" refers to optionally substituted saturated cyclic hydrocarbon ring systems, preferably containing 3 to 7 carbons per ring, or from 3 to 12 carbon atoms per ring system. Exemplary groups include cyclopropyl, cyclobutyl, cyclopentyl, cyclohexyl, cycloheptyl, cycooctyl, cyclodecyl, cyclododecyl and adamantly. Exemplary substituents include one or more alkyl groups as described above.

The term "alkoxyl" refers to straight and branched aliphatic hydrocarbon chains attached to an oxygen atom, for example methoxy, ethoxy, n-propoxy, isopropoxy and the like.

The term "aryl" refers to a C-radical derived from one of the known 1-4 aromatic ring systems, the rings being isolated or partially/totally fused and having 5-6 members; being each member independently selected from C, CH, N, NH, O, and S, being one or more of the hydrogen atoms of said members optionally substituted by radicals selected from the group consisting of lineal or branched (C₁-C₆)-alkyl, optionally substituted by halogen atoms; lineal or branched (C₁-C₆)-alkenyl optionally substituted by an halogen atom; lineal or branched (C₁-C₆)-alkoxyl optionally substituted by an halogen atom; halogen; aldehydes (CHO); ketones of formula CORₐ wherein Rₐ is radical selected from lineal or branched (C₁-C₆)-alkyl and phenyl; amines of formula NRₐR_{b}, wherein Rₐ and R_{b} are lineal or branched (C₁-C₆)-alkyl; ethers of formula ORₑ, wherein Rₑ is a lineal or branched (C₁-C₆)-alkyl or a tetrahydropyranyl group; thioethers of formula SR_{c} wherein R_{c}, is a lineal or branched (C₁-C₆)-alkyl; esters of formula COOR_{d}, wherein R_{d} is a lineal or branched (C₁-C₆)-alkyl; and epoxides of formula CH₂CHOCHRₐwherein Rₐ is a lineal or branched (C₁-C₆)-alkyl. Examples of aryls included, but are not limited to monocyclic, bicyclic or tricyclic aromatic ring system, for example phenyl, 1-naphthyl, 2-naphthyl, thiophenyl, indolyl and dioxole rings fused to the benzyl, napthyl, thiophenyl and indolyl ring, which may be substituted with one or more alkyl, alkoxyl, halo, or amino groups, as well as with one or more ethers, esters or amides of said groups.

In the sense of the present invention, an aromatic ring system includes simple aromatic carbon rings, also known as simple arenes or simple aromatics, and heteroaryl rings. The term "heteroaryl" refers to aromatic heterocyclic groups. Heteroaryl rings are tipically 5- or 6-membered aromatic rings containing one or more O, N, or S atoms which may be fused to one or more aromatic, heteroaromatic or heterocyclic rings such as a benzene ring. Examples of heteroaryl groups include but are not limited to, thienyl, furyl, pyrrolyl, triazolyl, thiazolyl, oxazolyl, isoaxolyl, thiazolyl, isothiazolyl, pyridyl, and pyrimidinyl.

When throughout the description the term "aryl halide" is used, it is to be understood any aryl compound which at least is substituted by one halogen atom. Said at least one halogen atom is the one which will be removed by CO₂ during the reaction conducted in the process of the invention.

A "benzoic acid derivative" refers to any compound having at least one benzene substituted with at least one carboxylic group, as well as any salt, ester or ether of said carboxylic group. The benzene ring may be fused with at least other aromatic or non-aromatic ring, and/or substituted with one or more alkyl, alkoxyl, halo, amino or thiol groups, as well as with one or more ethers, thioethers, esters, thioesters, thioamides or amides of said groups.

In the meaning of the present invention a "catalyst system" is the minimal group of compounds which in combination allows a reaction to be catalyzed. The combination of the compounds may act as a covalently bound structure or as independent compounds acting in an associated manner, simultaneously or sequentially.

The term "organozinc derivative" refers to any compound having as a core a zinc atom linked to an organic compound, preferably a lineal or branched (C₁-C₆)-alkan, and lineal or branched (C₁-C₆)-alkene.

The process of carboxylation of the invention is preferably conducted with a palladium compound selected from the group consisting of: Pd(OAc)₂, PdCl₂, PdBr₂, [PdCI(allyl)]₂, [PdCI(cinnamyl)]₂ and PdCl₂(acetonitrile)₂. Among all of them, the Pd(OAc)₂ is preferred.

Another preferred embodiment includes the process for the carboxylation of an aryl halide with CO₂ in which the compound of formula (I), also named ligand, is defined as the one in which R₁ is (C₁-C₆)-alkyl; R₂, R₃ and R₄ are (C₁-C₆)-alkyl; and R₅, R₆, R₇ and R₈ are H.

In another preferred embodiment R₁ is *tert-butyl;* R₂, R₃ and R₄ are isopropyl; and R₅, R₆, R₇ and R₈ are H.

The process for the carboxylation of an aryl halide with CO₂ according to the invention, includes as the preferred catalyst system Pd(OAc)₂ and a compound of formula (I) as defined in which R₁ is tert-butyl; R₂, R₃ and R₄ are isopropyl; and R₅, R₆, R₇ and R₈ are H.

As above exposed the process is conducted under inert atmosphere in an organic solvent and in the presence of a reducing agent. One of the preferred reducing agents is an organozinc derivative, being the preferred organozinc derivative the diethylzinc, Zn(CH₂CH₃)₂.

Accordingly, in yet another embodiment the organic solvent comprises at least a solvent selected from the group consisting of dimethylacetamide and dimethylformamide.

The process for the carboxylation of an aryl halide with CO₂ according to the invention may be preferably applied to aryl halides comprising at least one aromatic ring system with 5-6 members. The members of the aromatic ring system are independently selected from C, CH, N, NH, O, and S, and one or more of the H atoms of said members are optionally substituted by radicals selected from the group consisting of: lineal or branched (C₁-C₆)-alkyl, optionally substituted by halogen atoms; lineal or branched (C₁-C₆)-alkenyl optionally substituted by an halogen atom; lineal or branched (C₁-C₆)-alkoxyl optionally substituted by an halogen atom; halogen; aldehydes (CHO); ketones of formula CORₐ, wherein Rₐ is a radical selected from lineal or branched (C₁-C₆)-alkyl and phenyl; amines of formula NRₐR_{b}, wherein Rₐ and R_{b} are lineal or branched (C1-C₆)-alkyl; ethers of formula ORₑ, wherein Rₑ is a lineal or branched (C₁-C₆)-alkyl or a tetrahydropyranyl group; thioethers of formula SR_{c} wherein R, is a lineal or branched (C1-C₆)-alkyl; esters of formula COOR_{d}, wherein R_{d} is a lineal or branched (C1-C₆)-alkyl; and epoxides of formula CH₂CH OCHRₐwherein Rₐ is a lineal or branched (C₁-C₆)-alkyl.

It is also an embodiment of the invention, a process wherein the aryl halide comprises two aromatic ring systems with 5-6 members, being said aromatic ring systems partially or totally fused. Preferred two aromatic ring systems with 5-6 members include naphthalene.

In a preferred embodiment, the process is conducted with an aryl bromide, being the at least one aromatic ring system of said aryl bromide unsubstituted, partially substituted or totally substituted by an halogen atom. Indeed, as will be illustrated bellow, the presence of further halogen atoms in the molecule to be carboxylated using CO₂ does not alter the yield of the reaction. When aryl bromides are used, the bromide atom is the halogen preferably removed by CO₂ during the reaction conducted in the process of the invention. High yields of carboxylation are achieved with aryl bromides selected from the group consisting of: p-n-butyl-bromobenzene, 1-bromonaphtalene and o-methyl-bromobenzene.

Although the process can be done in a wide range of concentrations of the reagents and catalyst system, and in a wide range of temperatures, preferred amounts of the palladium compound in molar percentage are from 5 mol % to 20 mol %, preferred amounts of the compound of formula (I) in molar percentage are from 10 mol % to 40 mol %, preferred final concentrations for the reaction of the selected organic solvent are from 0.01 M to 0.5 M, preferred amounts of aryl halide range from 0.5 mmol to 10 mmol the organozinc derivative is added in the reaction pool with a range from 1.0 mmol to 20 mmol in the solvent, preferably 1.0 M, preferred CO₂ pressures for conducting the reaction range from 1 to 15 atm, and the process is carried out from 0 °C to 60 °C. Advantageously, the process of the invention may also be conducted at room temperature (18 °C to 30 °C) without significatively affecting the yield of the carboxylation reaction.

With the aim of further illustrating the process for the carboxylation of an aryl halide with CO₂ according to the invention, some examples are included herewith.

### EXAMPLES

### Example 1. Reaction and yields in different organic solvents.

The present example discloses the conversion of a non-activated aryl bromide (1 a) (p-n-butyl-bromobenzene) with CO₂ to obtain the corresponding benzoic acid (2a). In order to render the process catalytic, diethylzinc was used as the reducing agent. The employed catalyst system comprised the palladium diacetate (Pd(OAc)₂) with a compound of formula (I) with R₁ = *tert-*butyl; R₂, R₃ and R₄ = isopropyl; and R₅, R₆, R₇, and R₈= H.
In all the studied cases, significant amounts of dehalogenated and Negishi-type products, (3) and (4), respectively, were observed.

The reaction is schematically illustrated below: 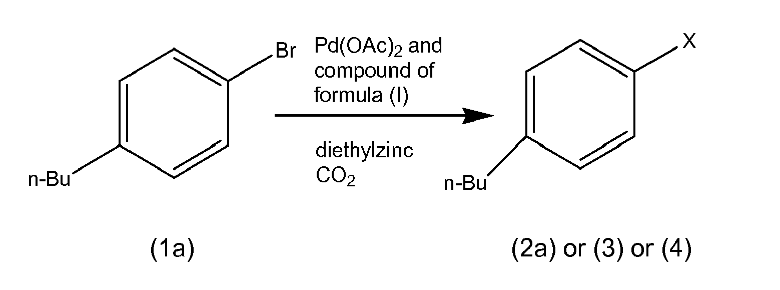 wherein X is CO₂H (product type 2a - corresponding benzoic acid), or H (product type 3), or ethyl (product type 4 ― Negishi-type products)

To perform the reaction 0.5 mmol of (1 a) were employed, the molar percentage of Pd(OAc)₂ was 5.0 mol %, the compound of formula (I) as above defined was used in a molar percentage of 10 mol %. The pressure of CO₂ was of 1 atm or 10 atm.

Following Table 1 lists different process conditions applied to the reaction illustrated above. The percentage by weight of the achieved products measured by gas chromatography using dodecane as internal standard, is shown:

**Table 1:**

| Entry | CO₂ pressure (atm) | Solvent | 2a (weight %) | 3 (weight %) | 4 (weight %) |
|---|---|---|---|---|---|
| 1 | 1 | DMA | 56 | 24 | 0 |
| 2 | 1 | DMF | 19 | 28 | 27 |
| 3 | 1 | NMP | 0 | 99 | 0 |
| 4 | 1 | DMSO | 0 | 31 | 69 |
| 5 | 1 | DMA | 35 | 24 | 0 |
| 6 | 10 | DMA | 64 | 17 | 0 |

| | | | | | |
|---|---|---|---|---|---|
| DMA: dimethylacetamide; DMF: dimethylformamide; NMP: N-methylpyrrolidone; DMSO: dimethylsulfoxide. | | | | | |

As can be deduced from Table 1, the nature of the organic solvent has a crucial impact on the reaction outcome. Thus, while dimethylacetamide (DMA) and dimethylformamide (DMF) clearly favored the carboxylation, N-methylpyrrolidone (NMP) or dimethylsulfoxide (DMSO) resulted in the exclusive formation of dehalogenated (3) and Negishi-type products (4). It is worth mentioning that the use of diethylacetamide (DEA), structurally related to DMA, led to the preferential formation of Negishi-type products (4), hence indicating the subtleties of this system (data not shown). Substitution of the organozinc by triethylaluminium or triethylsilane, among others, had also a deleterious effect, affording preferentially dehalogenated and Negishi-type products, (3) and (4) as final products (data not shown).

Entry 5 shows the results using diisopropylzinc (2.0 equivalents) as organozinc compound. As can be seen, the yield of the corresponding carboxylic acid obtained is lower than the corresponding with the same solvent (DMA) but using diethylzinc (Entry 1 of table 1), thus being diethylzinc the preferred organozinc reagent. Nonetheless, the best results were finally obtained by operating at a higher CO₂ pressure (10 atm), thus leading to formation of the benzoic acid (2a) in a 64 % isolated yield (entry 6 of table).

### Example 2. Palladium-catalyzed carboxylations of aryl bromides withCO₂ Observation of reagent effect.

With the preferred conditions selected from the experimentation as disclosed in Example 1, a wide range of substituted aryl bromides bearing both electron-donating and electron-withdrawing groups were tested. Table 2 shows the yields obtained by different reagents running the following reaction:

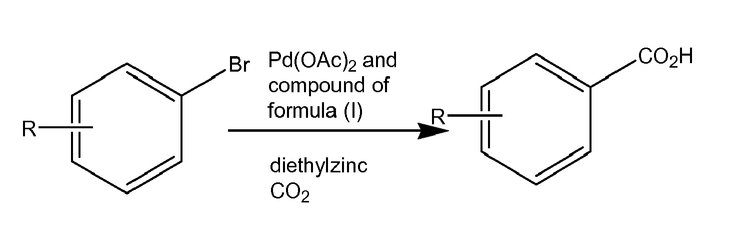

Wherein R represents any of the substituents listed in the final products of Table 2.

### Conditions:

CO₂ pressure = 10 atm
Pd(OAc)₂ molar percentage = 5 mol %
Compound of formula (I) with R₁ = tert-butyl; R₂, R₃ and R₄ = isopropyl; and
R₅, R₆, R₇, and R₈ = H; molar percentage of compound of formula (I) = 10 mol %
Organozinc derivative = diethylzinc (1.0 mmol, 1 M in hexanes)
Initial amount of aryl halide = 0.5 mmol or 1.5 mmol
Solvent: DMA / Hexanes (2:1)
Temperature = 40 °C

**Table 2:**

| Entry | Final product | Yield (weight % of final product) |
|---|---|---|
| 1 | 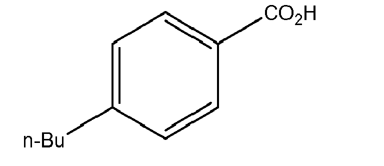 | 64 or 82 |
| 2 | 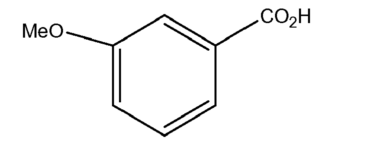 | 62 |
| 3 | 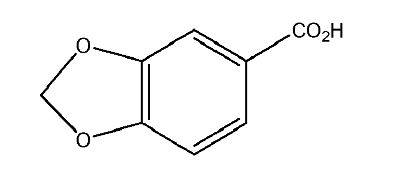 | 68 |
| 4 | 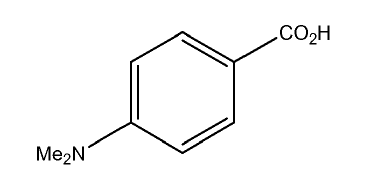 | 40 |
| 5 | 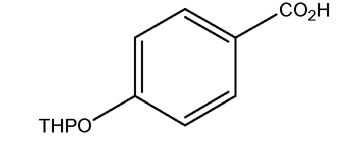 | 70 |
| 6 | 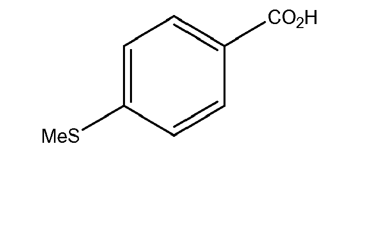 | 68 |
| 7 | 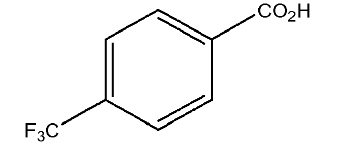 | 43 |
| 8 | 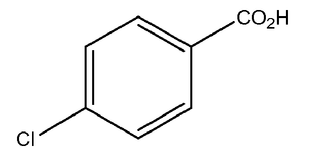 | 68 |
| 9 | 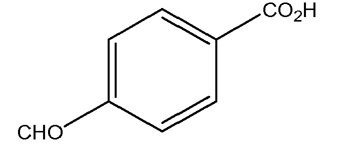 | 63 |
| 10 | 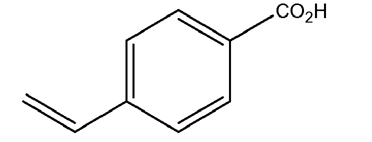 | 64 |
| 11 | 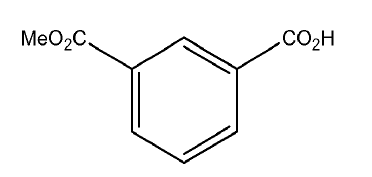 | 57 |
| 12 | 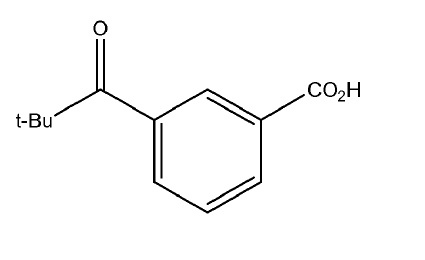 | 67 |
| 13 | 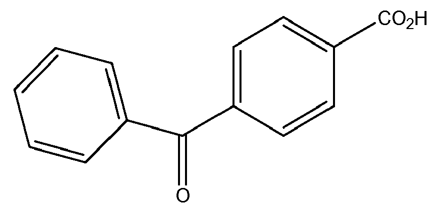 | 40 |
| 14 | 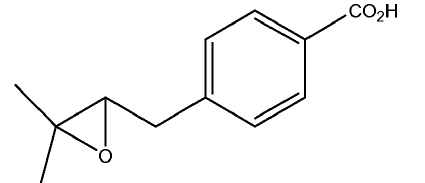 | 50 |
| 15 | 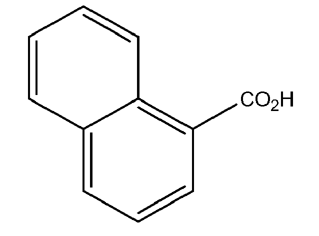 | 72 |
| 16 | 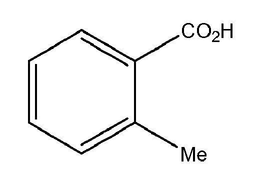 | 72 |
| 17 | 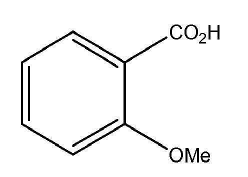 | 63 |
| 18 | 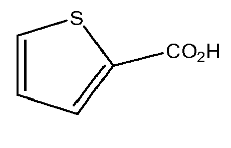 | 67 |
| 19 | 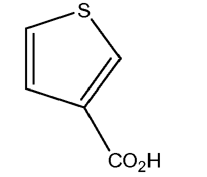 | 61 |

THPO: Tetrahydropyranyl which corresponds to the formula

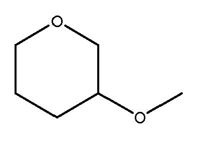

All of the aryl bromides listed in Table 2 smoothly underwent the target carboxylation delivering the corresponding benzoic acids in moderate to good yields (40-82 %, Table 2). Listed yields represent an average of at least two runs and it is expressed as the weight percentage obtained of the desired final product (isolated yield). The chemoselectivity of this novel transformation was clearly demonstrated by the tolerance of functional groups such as amines (entry 4), ethers (entry 5), thioethers (entry 6), alkenes (entry 10), esters (entry 11) and also heterocycles (entries 18 and 19). Additionally, ketones (entries 12 and 13) and even oxiranes (entry 14) remained inert under these reaction conditions, thus representing an additional bonus when comparing with the classical protocols involving the use of Grignard reagents or organolithiums. Moreover, the carboxylation reaction can be achieved in the presence of an aryl chloride, thus leaving options for subsequent manipulation (entry 8). Interestingly, the process was not hampered by ortho substituents (entries 16 and 17).

Entry 1 lists two yields of the reaction. A yield of 64 % was obtained when 0.5 mmol (milimoles) of the compound of entry 1 (p-n-butyl-bromobenzene) were used. When initially 1.5 mmol of the same compound were used the yield raised to the 84 % by weight.

All these examples allow to state that the process of the present invention, for the carboxylation of an aryl halide with CO₂ as the sole source of carbon, supposes a great improvement to other reactions with other catalyst systems and reactants. In fact, there is no need to prepare those corresponding organometallic intermediates broadly used in the prior art processes, thus constituting an additional bonus in practical and economical terms.

At the same time, the use of CO₂ (abundant, cheap, non-toxic and high potential as a renewable source) as the sole source of carbon is advantageous over those processes in which CO is employed.

Therefore, the catalyst system comprising a palladium compound and a compound of formula (I) is advantageously proposed for use in the carboxylation reactions in which CO₂ is added to an organic compound, namely a halogenated aromatic compound, to yield the corresponding carboxylic acid of said compound.

## Claims

1. A process for the carboxylation of an aryl halide to yield an aryl carboxylic acid, comprising contacting the aryl halide and CO₂ in an organic solvent under inert atmosphere and in the presence of a reducing agent and a catalyst system, said catalyst system comprising a palladium compound and a compound of formula (I) 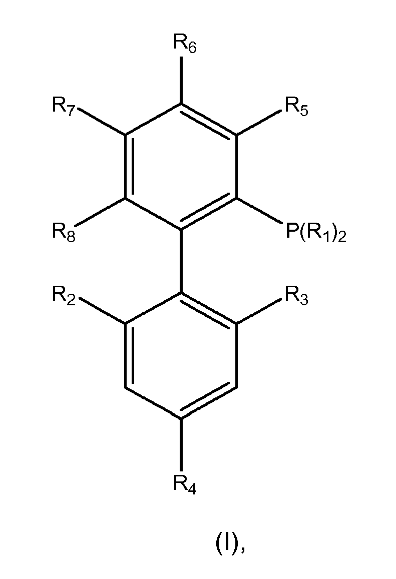 wherein R₁ is a radical independently selected from the group consisting of lineal or branched (C₁-C₆)-alkyl and (C₁-C₁₂)-cycloalkyl;
R₂ and R₃ are radicals independently selected from the group consisting of lineal or branched (C₁-C₆)-alkyl and lineal or branched (C₁-C₆)-alkoxyl;
R₄ is a lineal or branched (C₁-C₆)-alkyl radical;
R₅ is a radical independently selected from the group consisting of H, lineal or branched (C₁-C₆)-alkyl, and lineal or branched (C₁-C₆)-alkoxyl;
R₆, R₇ and R₈ are radicals independently selected from the group consisting of H and lineal or branched (C₁-C₆)-alkyl; and
"aryl" is a C-radical derived from one of the known 1-4 aromatic ring systems, the rings being isolated or partially/totally fused and having 5-6 members; being each member independently selected from C, CH, N, NH, O, and S, being one or more of the hydrogen atoms of said members optionally substituted by radicals selected from the group consisting of lineal or branched (C₁-C₆)-alkyl, optionally substituted by halogen atoms; lineal or branched (C₁-C₆)-alkenyl, optionally substituted by an halogen atom; lineal or branched (C₁-C₆)-alkoxyl, optionally substituted by an halogen atom; halogen; aldehydes (CHO); ketones of formula CORₐ wherein Rₐ is radical selected from lineal or branched (C₁-C₆)-alkyl and phenyl; amines of formula NRₐR_{b}, wherein Rₐ and R_{b} are lineal or branched (C₁-C₆)-alkyl; ethers of formula ORₑ, wherein Rₑ is a lineal or branched (C₁-C₆)-alkyl or a tetrahydropyranyl group; thioethers of formula SR_{c} wherein R_{c} is a lineal or branched (C₁-C₆)-alkyl; esters of formula COOR_{d}, wherein R_{d} is a lineal or branched (C₁-C₆)-alkyl; and epoxides of formula CH₂CH OCHRₐwherein Rₐ is a lineal or branched (C₁-C₆)-alkyl.

2. The process of claim 1, wherein the palladium compound is selected from the group consisting of: Pd(OAc)₂, PdCl₂, PdBr₂, [PdCI(allyl)]₂, [PdCI(cinnamyl)]₂ and PdCl₂(acetonitrile)₂

3. The process of claim 1, wherein the palladium compound is Pd(OAc)₂.

4. The process according to any of the claims 1-3, wherein R₁ is (C₁-C₆)-alkyl; R₂, R₃ and R₄ are (C₁-C₆)-alkyl; and R₅, R₆, R, and R₈ are H.

5. The process according to claim 4, wherein R₁ is tert-butyl;R₂, R₃ and R₄ are isopropyl; and R₅, R₆, R₇ and R₈ are H.

6. The process according to any of the claims 1-5, wherein the reducing agent is an organozinc derivative.

7. The process according to claim 6, wherein the organozinc derivative is diethylzinc Zn(CH₂CH₃)₂.

8. The process according to any of the claims 1-7, wherein the organic solvent comprises at least a solvent selected from the group consisting of: dimethylacetamide and dimethylformamide.

9. The process according to any of the claims 1-8, wherein the aryl halide comprises at least one aromatic ring system with 5-6 members, being said members independently selected from C, CH, N, NH, O, and S, and being one or more of the H atoms of said members optionally substituted by radicals selected from the group consisting of: lineal or branched (C₁-C₆)-alkyl, optionally substituted by halogen atoms; lineal or branched (C₁-C₆)-alkenyl optionally substituted by an halogen atom; lineal or branched (C₁-C₆)-alkoxyl optionally substituted by an halogen atom; halogen; aldehydes (CHO); ketones of formula CORₐ wherein Rₐ is radical selected from lineal or branched (C₁-C₆)-alkyl and phenyl; amines of formula NRₐR_{b}, wherein Rₐ and R_{b} are lineal or branched (C₁-C₆)-alkyl; ethers of formula ORₑ, wherein Rₑ is a lineal or branched (C₁-C6)-alkyl or a tetrahydropyranyl group; thioethers of formula SR_{c}, wherein R_{c} is a lineal or branched (C₁-C₆)-alkyl; esters of formula COOR_{d}, wherein R_{d} is a lineal or branched (C₁-C₆)-alkyl; and epoxides of formula CH₂CH OCHRₐwherein Rₐ is a lineal or branched (C₁-C₆)-alkyl.

10. The process according to claim 9, wherein the aryl halide comprises two aromatic ring systems with 5-6 members, being said aromatic ring systems partially or totally fused.

11. The process according to any of the claims 1-10, wherein the aryl halide is an aryl bromide.

12. The process according to any of the claims 1-11, wherein the palladium compound is provided in a range of molar percentage from 5 mol % to 20 mol %.

13. The process according to any of the claims 1-12, wherein the compound of formula (I) is provided in a range of molar percentage from 10 mol % to 40 mol %.

14. The process according to any of the claims 1-13, wherein the aryl halide is provided in a amount ranging from 0.5 to 10 milimoles (mmol).

15. The process according to any of the claims 1-14, wherein CO₂ is provided in a pressure ranging from 1 to 15 atm.
